# EUROPÄISCHE PATENTSCHRIFT

(11) **EP 2 297 132 B1**
(45) Veröffentlichungstag und Bekanntmachung des Hinweises auf die Patenterteilung: **25.02.2015**
(21) Anmeldenummer: 09765795.1
(22) Anmeldetag: 12.06.2009
(51) Int. Cl.: C07D 401/12

(54) **VERFAHREN ZUR HERSTELLUNG EINES ZWISCHENPRODUKTS VON DABIGATRAN ETEXILATE**
METHOD FOR MANUFACTURING AN INTERMEDIARY PRODUCT OF DABIGATRAN ETEXILATE
PROCÉDÉ POUR LA RÉALISATION D'UN PRODUIT INTERMÉDIAIRE DE DABIGATRAN ETEXILATE

(30) Priorität: 16.06.2008 EP 08158364
(43) Veröffentlichungstag der Anmeldung: 23.03.2011
(73) Patentinhaber: Boehringer Ingelheim International GmbH, 55216 Ingelheim am Rhein (DE)
(72) Erfinder: DERSCH, Wolfgang, 55216 Ingelheim am Rhein (DE); FILSER, Christian, 55216 Ingelheim am Rhein (DE); HAMM, Rainer, 55216 Ingelheim am Rhein (DE); HAUSHERR, Arndt, 55216 Ingelheim am Rhein (DE); KOCH, Gunter, 55216 Ingelheim am Rhein (DE); SCHOLZ, Ulrich, 55216 Ingelheim am Rhein (DE); ZERBAN, Georg, 55216 Ingelheim am Rhein (DE)
(74) Vertreter: Simon, Elke Anna Maria
(86) Internationale Anmeldenummer: PCT/EP2009/057266
(87) Internationale Veröffentlichungsnummer: WO 2009/153215

(56) Entgegenhaltungen:
- WO-A-2006/000353
- WO-A-2007/071742

## Beschreibung

Die Erfindung betrifft ein Verfahren zur Herstellung der Verbindung der Formel **1** 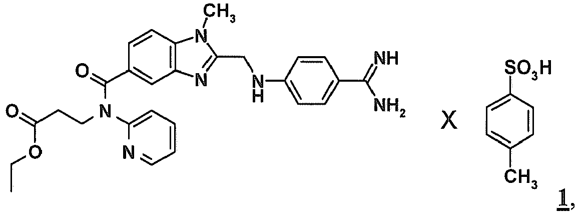 einem wertvollen Zwischenprodukt bei der Synthese des pharmazeutischen Wirkstoffs Dabigatranetexilat.

### Stand der Technik

Dabigatranetexilat ist im Stand der Technik bekannt und wurde erstmals durch die internationale Patentanmeldung WO 98/37075 offenbart. Verfahren zur Herstellung des Dabigatranetexilats sind ferner bekannt aus der WO 2006/000353 oder auch aus Hauel et al. (J. Med. Chem, 2002, 45, 1757 ff).

Wie aus der WO 98/37075 oder auch der WO 2006/000353 erkennbar, kommt der Verbindung der Formel **1**, dem 1-Methyl-2-[*N*-[4-amidinophenyl]-amino-methyl]-benzimidazol-5-yl-carboxylsäure-*N*-(2-pyridyl)-*N*-(2-ethoxycarbonylethyl)-amid-p-Toluolsulfonsäuresalz, bei der Synthese des Dabigatranetexilats eine zentrale Bedeutung als Zwischenprodukt zu.

Neben den Internationalen Patentanmeldungen WO 98/37075 und WO 2006/000353 offenbaren auch die WO 2007/071742 A1 und WO 2007/071743 A1 Aspekte möglicher Herstellverfahren des Dabigatranetexilats.

In der WO 98/37075 wird vorgeschlagen, die substituierten (4-Benzimidazol-2-ylmethylamino)-benzamidine durch Umsetzung der entsprechenden substituierten (4-Benzimidazol-2-ylmethylamino)-benzonitrile mit Ammoniak herzustellen. Dieses Verfahren ist produktionstechnisch sehr aufwändig und führt zu einer hohen Belastung an zu entsorgenden Säuren.

In den Patentanmeldungen WO 2006/000353 A1, WO 2007/071742 A1 und WO 2007/071743 A1 erfolgt die Herstellung der Verbindung der Formel **1** über die Synthese des Kondensationsprodukts der Formel **4**, wie sie im nachfolgenden Schema 1 dargestellt ist.

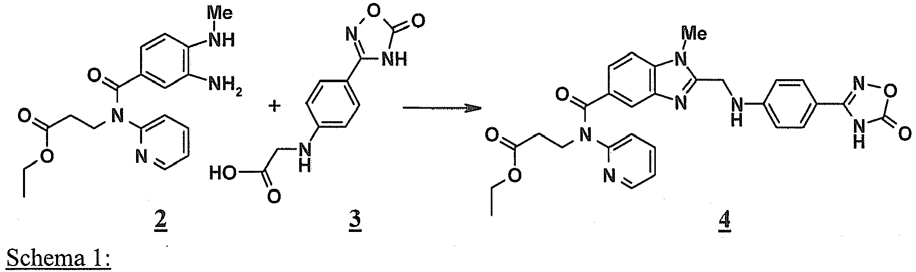

Die Verbindung der Formel **4** wird gemäß den im Stand der Technik beschriebenen Verfahren zunächst isoliert und anschließend hydriert.

Bei der Kondensation gemäß Schema 1 tritt häufig das Nebenprodukt der Formel **5** auf, welches vor der Isolierung des Kondensationsproduktes **4** in einer aufwendigen Heißfiltration entfernt werden muss.

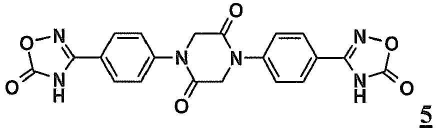

Darüberhinaus erfordert die weitergehende Umsetzung zur Verbindung der Formel **1** einen Lösungsmittelwechsel und zudem eine sehr zeit- und kostenaufwendige Isolierung und Trocknung der Zwischenstufe **4**. Diese kann mit hohen Ausbeuteverlusten verbunden sein.

Es ist Aufgabe der vorliegenden Erfindung ein Verfahren bereitzustellen, welches die großtechnische Synthese der Verbindung der Formel **1** in verbesserter Art und Weise erlaubt und bei der die vorstehend genannten Nachteile vermieden werden können.

### Detaillierte Beschreibung der Erfindung

Die vorliegende Erfindung betrifft ein Verfahren zur großtechnischen Herstellung der Verbindung der Formel 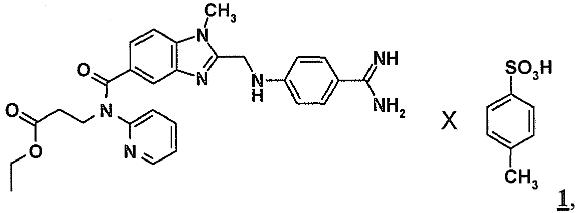 dadurch gekennzeichnet, dass ein Diamin der Formel **2** 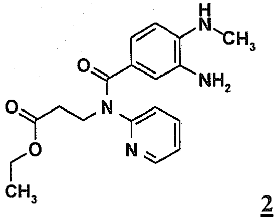 durch Umsetzung mit einem Oxadiazolon der Formel **3** 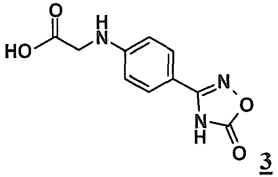 zu einer Verbindung der Formel **4** 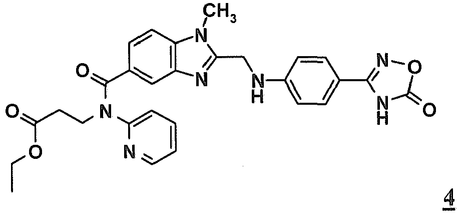 umgesetzt wird, die ohne Isolierung durch Hydrierung und Zugabe von p-Toluolsulfonsäure und Ammoniak in das Amidin der Formel **1** umgewandelt wird.

Die Darstellung der Ausgangsverbindungen der Formeln **2** und **3** kann gemäß der in der WO 2006/000353 erläuterten Vorgehensweise erfolgen.

Zur erfindungsgemäßen Umsetzung werden **2** und **3** in einem inerten, organischen Lösungsmittel gelöst und in Gegenwart eines wasserbindenden Mittels kondensiert.

Als inertes, organisches Lösungsmittel kommen bevorzugt aprotische Lösungsmittel in Betracht. Diese sind beispielsweise ausgewählt aus der Gruppe bestehend aus aliphatische oder aromatische, gegebenenfalls halogenierte Kohlenwasserstoffe, Ether, Amide oder Gemische davon. Als aprotische apolare Lösungsmittel werden bevorzugt verzweigte oder unverzweigte C₅ - C₈ aliphatische Alkane, C₄ - C₁₀ Cycloalkane, C₁- C₆ aliphatische Halogenalkane, C₆ - C₁₀ aromatische Alkane oder Gemische davon eingesetzt. Besonders bevorzugt werden Alkane wie Pentan, Hexan oder Heptan, Cycloalkane wie Cyclohexan oder Methylcyclohexan, Halogenalkane wie Dichlormethan, aromatische Alkane wie Benzol, Toluol oder Xylol oder deren Mischungen eingesetzt. Als aprotische Lösungsmittel eignen sich ferner polare Ether wie beispielsweise Tetrahydrofuran (THF), Methyltetrahydrofuran, Dioxan, *tert*-Butyl-Methylether oder Dimethoxyethylether oder Amide wie beispielsweise Dimethylformamid, oder Lactame wie beispielsweise N-Methylpyrrolidon.

Als wasserbindende Mittel können hygroskopische Salze, anorganische oder organische Säuren bzw. deren Säurechloride, Anhydride von anorganischen oder organischen Säuren, Anhydride von Alkanphosphonsäuren, Molsiebe oder Harnstoffderivate eingesetzt werden. Bevorzugt sind 1,1'-Carbonyldiimidazol und Alkanphosphonsäureanhydride, besonders bevorzugt sind Alkanphosphonsäureanhydride. Bei letzteren kommt dem Propanphosphonsäureanhydrid (PPA = 2,4,6-Tripropyl-[1,3,5,2,4,6]trioxatriphosphinane 2,4,6-trioxid) erfindungsgemäß besondere Bedeutung zu.

Gelangen Alkanphosphonsäureanhydride zur Anwendung wird erfindungsgemäß bevorzugt eine organische Base, besonders bevorzugt ein tertiäres Amin, insbesondere bevorzugt Diisopropylethylamin zugesetzt.

Pro Mol eingesetzte Verbindung der Formel **2** werden bevorzugt 0,5 - 2,5 1 (Liter), besonders bevorzugt 1,0 - 2,01, ferner bevorzugt 1,3 - 1,5 l des vorstehend genannten inerten organischen Lösungsmittels verwendet.

Pro Mol eingesetzte Verbindung der Formel **2** werden bevorzugt wenigstens stöchiometrische Mengen der Verbindung der Formel **3** eingesetzt. Besonders bevorzugt wird die Verbindung der Formel **3** in leichtem Überschuß eingesetzt. Pro Mol eingesetzte Verbindung der Formel **2** werden 1,0 - 2,0 Mol, besonders bevorzugt 1,0 - 1,5 Mol besonders bevorzugt 1,1 - 1,3 Mol der Verbindung der Formel **3** verwendet.

Die Verbindungen **2** und **3** werden im vorstehend genannten inerten, organischen Lösungsmittel bei 10 - 50°C, bevorzugt bei 20 - 40°C, besonders bevorzugt bei 25 - 35°C gelöst. Bei konstanter Temperatur wird anschließend in der erfindungsgemäß besonders bevorzugten Ausführungsform das tertiäre Amin zugegeben. Pro Mol eingesetzte Verbindung der Formel **2** werden bevorzugt wenigstens stöchiometrische Mengen des tertiären Amins eingesetzt. Besonders bevorzugt wird das tertiäre Amin allerdings in großem Überschuß verwendet. Pro Mol eingesetzte Verbindung der Formel **2** gelangen dementsprechend 1,5 - 5,0 Mol, besonders bevorzugt 2,0 - 4,0 Mol besonders bevorzugt 2,3 - 2,7 Mol des tertiären Amins zur Anwendung.

Nach beendeter Zugabe des tertitären Amins wird vorzugsweise bei einer Temperatur im Bereich von 10 - 40°C, besonders bevorzugt bei 20-30°C das Alkanphosphonsäureanhydrid, bevorzugt PPA zudosiert. Pro Mol eingesetzte Verbindung der Formel **2** werden bevorzugt wenigstens stöchiometrische Mengen des Alkanphosphonsäureanhydrids eingesetzt. Besonders bevorzugt wird das Alkanphosphonsäureanhydrid in leichtem Überschuß eingesetzt. Pro Mol eingesetzte Verbindung der Formel **2** werden besonders bevorzugt 1,0 - 2,0 Mol, besonders bevorzugt 1,0 - 1,7 Mol besonders bevorzugt 1,1 - 1,4 Mol des Alkanphosphonsäureanhydrids verwendet. Das erfindungsgemäß bevorzugt zum Einsatz gelangende PPA wird bevorzugt in verdünnter Form zugesetzt. In einer bevorzugten Ausführungsform wird es zur Zugabe in dem zum Einsatz gelangenden inerten, organischen Lösemittel aufgenommen. Besonders bevorzugt erfolgt die Zugabe des PPA in 30 - 60 %-iger (Gew-%), vorzugsweise in 50%-iger Lösung in Tetrahydrofuran oder Essigsäureethylester.

Nach beendeter PPA Zugabe wird bei konstanter Temperatur für etwa 0,25 - 4 h nachgerührt. Sodann werden bezogen auf eingesetzte Verbindung **2** vorzugsweise wenigstens 0,5 Äquivalente einer schwachen organischen Säure zugesetzt. Bei der schwachen organischen Säure handelt es sich vorzugsweise um Zitronen- oder Essigsäure. Die Säure kann allerdings auch im Überschuß verwendet werden. Pro Mol eingesetzte Verbindung der Formel **2** gelangen dementsprechend 0,5 - 4,0 Äquivalente, besonders bevorzugt 1,0 - 3 Äquivalents, besonders bevorzugt 1,0 - 2,0 Äquivalente der Säure zur Anwendung. Gegebenenfalls kann das Reaktionsgemisch mit einem der vorstehend genannten inerten organischen Lösungsmittel, vorzugsweise mit demselben Lösungsmittel, verdünnt werden. Zur Verdünnung werden bevorzugt bis zu 50%, besonders bevorzugt 10 - 30 % der bereits vorgelegten Lösungsmittelmenge hinzugefügt.

Nach Säurezugabe und gegebenenfalls Verdünnen wird die Kondensation zur Verbindung **4** bei erhöhter Temperatur und gegebenenfalls erhöhtem Druck durchgeführt. Die Temperatur wird erfindungsgemäß bevorzugt in einem Bereich von oberhalb von 50°C, bevorzugt bei 60 - 100°C, besonders bevorzugt bei 65-85°C gehalten. Sofern ein Lösungsmittel Verwendung findet, welches in diesem Bereich bereits siedet, wird der Druck so erhöht, dass trotz des niedrigeren Siedepunktes die Reaktion bei der genannten Temperatur geführt werden kann. Bevorzugt wird der Druck, bei dem die Reaktion geführt wird, auf einen Wert von 1-3 bar eingestellt.

Der Reaktionsverlauf wird nach üblichen Verfahren, beispielsweise mittels Dünnschichtchromatographie oder HPLC kontrolliert. Nach Umsetzung wird die Reaktionsmischung langsam abgekühlt, vorzugsweise auf eine Temperatur im Bereich von 10 - 50°C, besonders bevorzugt auf etwa 20-30°C.

Ohne weitergehende Aufarbeitung wird der Reaktionsmischung jetzt ein geeigneter Hydrier-Katalysator zugesetzt. Als Hydrierkatalysatoren sind in der Regel Übergangsmetalle wie zum Beispiel Nickel, Platin oder Palladium oder deren Salze oder Oxide geeignet. Bevorzugt sind Raney Nickel, Platinoxid und Palladium auf einem inerten Trägermaterial, insbesondere Palladium auf Aktivkohle (Pd/C).

In einer bevorzugten Ausführungsform wird wasserfeuchtes 10%-igem Pd/C verwendet. Pro Mol eingesetzte Verbindung der Formel **2** gelangen bevorzugt etwa 2 - 35 g, besonders bevorzugt etwa 4 - 25 g, besonders bevorzugt etwa 8 - 18 g dieses Katalysators zur Anwendung.

Nach Zugabe des Hydrierkatalysators wird Wasser zugesetzt. Die zugesetzte Wassermenge bestimmt sich bevorzugt nach der Gesamtmenge des verwendeten inerten, organischen Lösungsmittels. Vorzugsweise beträgt die zugesetzte Wassermenge 50 - 100% (v/v), besonders bevorzugt 70 - 90% (v/v) der eingesetzten Lösungsmittelgesamtmenge.

Nach Wasserzugabe wird die Reaktionsmischung auf eine Temperatur im Bereich von 30 - 70°C, besonders bevorzugt auf etwa 40-60°C erwärmt und unter Rühren bei einem Wasserstoffdruck von etwa 2 - 6 bar, bevorzugt 3 - 5 bar hydriert.

Nach der Umsetzung wird der Katalysator abfiltriert, das Filtrat gegebenenfalls mit etwa 5 - 30 % der zuvor verwendeten Wassermenge verdünnt und bei einer Temperatur im Bereich von 10 - 60°C, besonders bevorzugt auf etwa 20-40°C mit para-Toluolsulfonsäure versetzt. Die para-Toluolsulfonsäure kann als Feststoff, gegebenenfalls in Form ihres Monohydrats oder auch in wässeriger Lösung zugegeben werden. Pro Mol eingesetzte Verbindung der Formel **2** werden bevorzugt wenigstens stöchiometrische Mengen der para-Toluolsulfonsäure eingesetzt. Besonders bevorzugt wird die para-Toluolsulfonsäure in leichtem Überschuß eingesetzt. Pro Mol eingesetzte Verbindung der Formel **2** werden besonders bevorzugt 1,0 - 2,0 Mol, besonders bevorzugt 1,0 - 1,7 Mol, besonders bevorzugt 1,0 - 1,4 Mol, besonders bevorzugt 1,1 - 1,3 Mol der para-Toluolsulfonsäure verwendet.

Anschließend erfolgt die Zugabe von Ammoniak, entweder gasförmig oder in Form wässeriger Lösungen. Erfindungsgemäß bevorzugt wird der Ammoniak in Form wässeriger Lösungen eingesetzt, besonders bevorzugt in Form wässeriger Lösung mit einem Gehalt an etwa 25% (w/w) Ammoniak (NH₄OH). Die Zugabe kann beispielsweise bei einer Temperatur im Bereich von 30 - 65°C erfolgen. Pro mol eingesetzter Verbindung der Formel **2** werden an dieser Stelle bevorzugt 2 - 20 Mol, besonders bevorzugt 6 - 16 Mol, besonders bevorzugt etwa 9 - 13 mol Ammoniak zugesetzt.

Während der Ammoniakzugabe beginnt die Verbindung **1** auszukristallisieren. Die Reaktionsmischung wird auf eine Temperatur im Bereich von 0 - 40°C, besonders bevorzugt auf etwa 15-25°C abgekühlt, die Verbindung **1** abfiltriert und mit Wasser, oder Aceton gewaschen. Der Mutterlauge kann gegebenenfalls nochmals Ammoniak zugesetzt werden um weitere Verbindung **1** auszukristallieren. Wird weiterer Ammoniak zugegeben, sind dies pro mol eingesetzter Verbindung der Formel **2** an dieser Stelle bevorzugt 1 - 10 Mol, besonders bevorzugt 2 - 8 Mol, besonders bevorzugt etwa 3 - 5 mol Ammoniak.

Überraschenderweise wird durch Zugabe des Ammoniaks die Verbindung **1** aus der Reaktionsmischung fast vollständig gefällt. Dadurch ergeben sich im Vergleich zu den aus dem Stand der Technik bekannten Verfahren eine Reihe von Vorteilen, von denen einige nachstehend aufgeführt sind. Die Ausbeute an Verbindung **1** wird deutlich erhöht. Die gegebenenfalls zur Entfernung der Verunreinigung **5** erforderliche Heißfiltration bei der Herstellung des Intermediats **4** entfällt. Darüber hinaus werden weniger Lösungsmittel und Reagenzien benötigt, was insbesondere im technischen Maßstab die Durchführung der Synthese deutlich erleichtert. Darüber hinaus entfällt im Vergleich zum Stand der Technik die zeitaufwendige Isolierung und Trocknung einer Zwischenstufe.

Vor- und nachstehend werden folgende Abkürzungen verwendet:
AcOH Essigsäure
DIPEA*N*,*N*-Diisopropylethylamin
EtOAc Essigsäureethylester
Pd/C Palladium auf Aktivkohle
PPA Propanphosphonsäureanhydrid
PTSA p-Toluolsulfonsäure
RT Raumtemperatur
THF Tetrahydrofuran

Die nachfolgenden Beispiele dienen der Illustration eines exemplarisch durchgeführten Syntheseverfahrens. Sie sind lediglich als mögliche, exemplarisch dargestellte Vorgehensweise zu verstehen, ohne die Erfindung auf deren Inhalt zu beschränken.

### Beispiel 1:

24,20 g **2** und 19,95 g **3** werden in 100 ml THF bei ca. 30 °C weitgehend gelöst. Bei dieser Temperatur werden anschließend 24,92 g DIPEA zugegeben. Danach werden bei RT 57,89g einer 50%igen Lösung von PPA in THF zudosiert und ca. 2 h nachgerührt.

Nach Zugabe von 13,44 g Zitronensäure und 20 ml THF wird die Kondensation zum nicht isolierten Intermediat **4** bei ca. 90 °C unter Druck durchgeführt. Nach erfolgter Umsetzung wird die Reaktionsmischung auf RT abgekühlt und mit 1,21 g wasserfeuchtem 10 % Pd/C und 100 ml Wasser versetzt. Dann wird die Suspension auf ca. 50 °C erwärmt und unter einer Wasserstoffatmosphäre (bei ca. 4 bar) hydriert.

Pd/C wird abfiltriert und mit 25 ml Wasser gewaschen. Nach Zugabe von 25 ml Wasser wird die Reaktionsmischung bei ca. 50 °C mit 20,40 g einer 65%igen wässrigen PTSA-Lösung und 60 ml einer 25%igen wässrigen Ammoniaklösung versetzt. Dabei beginnt das Tosylat auszufallen. Es wird auf RT abgekühlt, das Produkt **1** abfiltriert und mit Wasser nachgewaschen.

Die Trocknung erfolgt bei 60 °C oder bis zu 95 °C im Vakuum.
Ausbeute: 41,4 g (87,2 %)
Reinheit: > 99 % HPLC-Peakfläche

### Beispiel 2:

24,20 g **2** und 19,95 g **3** werden in 87 ml THF bei RT weitgehend gelöst. Bei dieser Temperatur werden anschließend 24,92 g DIPEA zugegeben. Danach werden bei RT 57,89 g einer 50%igen Lösung von PPA in THF zudosiert, mit 13 ml THF nachgespült und ca. 2 h nachgerührt. Nach Zugabe von 6,72 g Zitronensäure und 20 ml THF wird die Kondensation zum nicht isolierten Intermediat **4** bei ca. 90 °C unter Druck durchgeführt.

Nach erfolgter Umsetzung wird die Reaktionsmischung auf RT abgekühlt und mit 1,24 g wasserfeuchtem 10 % Pd/C und 60 ml Wasser versetzt. Dann wird die Suspension auf ca. 50 °C erwärmt und unter einer Wasserstoffatmosphäre (bei ca. 4 bar) hydriert.

Pd/C wird abfiltriert und mit 50 ml eines THF-Wasser-Gemischs (7:3) gewaschen. Nach Zugabe von 20 ml eines THF-Wasser-Gemischs (7:3) wird die Reaktionsmischung bei ca. 50 °C mit 39,93 g fester PTSA und 60 ml einer 25%igen wässrigen Ammoniaklösung versetzt. Dabei beginnt das Tosylat auszufallen. Es wird auf RT abgekühlt, das Produkt **1** abfiltriert und mit Wasser nachgewaschen.

Die Trocknung erfolgt bei 40 °C oder bis zu 95 °C im Vakuum.
Ausbeute: 42,2 g (88,9 %); Reinheit: > 99 % HPLC-Peakfläche

### Beispiel 3:

24,20 g **2** und 19,95 g **3** werden in 87 ml THF bei RT weitgehend gelöst. Bei dieser Temperatur werden anschließend 24,92 g DIPEA zugegeben. Danach werden bei RT 57,89 g einer 50%igen Lösung von PPA in EtOAc zudosiert, mit 13 ml THF nachgespült und ca. 2 h nachgerührt. Nach Zugabe von 6,72 g Zitronensäure und 20 ml THF wird die Kondensation zum nicht isolierten Intermediat *BIBR 1048 Oxa-Amidin* bei ca. 90 °C unter Druck durchgeführt. Nach erfolgter Umsetzung wird die Reaktionsmischung auf RT abgekühlt und mit 1,21 g wasserfeuchtem 10 % Pd/C und 75 ml Wasser versetzt. Dann wird die Suspension auf ca. 50 °C erwärmt und unter einer Wasserstoffatmosphäre (bei ca. 4 bar) hydriert. Pd/C wird abfiltriert und mit 50 ml eines THF-Wasser-Gemischs (1:1) gewaschen. Nach Zugabe von 25 ml THF und 10 ml Wasser wird die Reaktionsmischung bei ca. 50 °C mit 39,93 g fester PTSA und 60 ml einer 25%igen wässrigen Ammoniaklösung versetzt. Dabei beginnt das Tosylat auszufallen. Es wird auf RT abgekühlt, das Produkt **1** abfiltriert und mit Wasser nachgewaschen.

Die Trocknung erfolgt bei 40 °C oder bis zu 95 °C im Vakuum.
Ausbeute: 43,1 g (90,8 %); Reinheit: > 99 % HPLC-Peakfläche

### Beispiel 4:

24,20 g **2** und 19,95 g **3** werden in 87 ml THF bei RT weitgehend gelöst. Bei dieser Temperatur werden anschließend 24,92 g DIPEA zugegeben. Danach werden bei RT 57,89 g einer 50%igen Lösung von PPA in EtOAc zudosiert, mit 13 ml THF nachgespült und ca. 2 h nachgerührt. Nach Zugabe von 4,20 g AcOH und 20 ml THF wird die Kondensation zum nicht isolierten Intermediat **4** bei ca. 90 °C unter Druck durchgeführt. Nach erfolgter Umsetzung wird die Reaktionsmischung auf RT abgekühlt und mit 1,25 g wasserfeuchtem 10 % Pd/C und 60 ml Wasser versetzt. Dann wird die Suspension auf ca. 50 °C erwärmt und unter einer Wasserstoffatmosphäre (bei ca. 4 bar) hydriert. Pd/C wird abfiltriert und mit 50 ml eines THF-Wasser-Gemischs (7:3) gewaschen. Nach Zugabe von 20 ml eines THF-Wasser-Gemischs (7:3) wird die Reaktionsmischung bei ca. 50 °C mit 39,93 g fester PTSA und 60 ml einer 25%igen wässrigen Ammoniaklösung versetzt. Dabei beginnt das Tosylat auszufallen. Es wird auf RT abgekühlt, das Produkt **1** abfiltriert und mit Wasser nachgewaschen. Die Trocknung erfolgt bei 45 °C oder bis zu 95 °C im Vakuum. Ausbeute: 36,4 g (76,7 %); Reinheit: > 99 % HPLC-Peakfläche

### Beispiel 5:

24,20 g **2** und 19,95 g **3** werden in 86 ml THF bei ca. 30 °C weitgehend gelöst. Bei dieser Temperatur werden anschließend 24,92 g DIPEA zugegeben. Danach werden bei RT 57,89 g einer 50%igen Lösung von PPA in THF zudosiert und ca. 2 h nachgerührt.

Nach Zugabe von 10,50 g L-Weinsäure und 20 ml THF wird die Kondensation zum nicht isolierten Intermediat **4** bei ca. 90 °C unter Druck durchgeführt. Nach erfolgter Umsetzung wird die Reaktionsmischung auf RT abgekühlt und mit 1,21 g wasserfeuchtem 10 % Pd/C und 100 ml Wasser versetzt. Dann wird die Suspension auf ca. 50 °C erwärmt und unter einer Wasserstoffatmosphäre (bei ca. 4 bar) hydriert. Pd/C wird abfiltriert und mit 30 ml Wasser gewaschen. Nach Zugabe von 20 ml Wasser wird die Reaktionsmischung bei ca. 50 °C mit 22,25 g einer 65%igen wässrigen PTSA-Lösung und 60 ml einer 25%igen wässrigen Ammoniaklösung versetzt. Dabei beginnt das Tosylat auszufallen. Es wird auf RT abgekühlt, das Produkt **1** abfiltriert und mit Wasser nachgewaschen.

Die Trocknung erfolgt bei 90 °C oder bis zu 95 °C im Vakuum.
Ausbeute: 39,3 g (82,8 %); Reinheit: > 99 % HPLC-Peakfläche.

### Beispiel 6:

24,20 g **2** und 19,95 g **3** werden in 100 ml THF bei ca. 30 °C weitgehend gelöst. Bei dieser Temperatur werden anschließend 23,07 g DIPEA zugegeben. Danach werden bei 25 °C 57,89g einer 50%igen Lösung von PPA in THF zudosiert und ca. 30 min nachgerührt. Nach Zugabe von 20,17 g Zitronensäure und 20 ml THF wird die Kondensation zum nicht isolierten Intermediat **4** bei ca. 75 °C unter Druck durchgeführt. Nach erfolgter Umsetzung wird die Reaktionsmischung auf RT abgekühlt und mit 1,21 g wasserfeuchtem 10 % Pd/C und 100 ml Wasser versetzt. Dann wird die Suspension auf ca. 50 °C erwärmt und unter einer Wasserstoffatmosphäre (bei ca. 4 bar) hydriert.

Pd/C wird abfiltriert und mit 30 ml Wasser gewaschen. Nach Zugabe von 20 ml Wasser wird die Reaktionsmischung bei 28-38 °C mit 22,25 g einer 65%igen wässrigen PTSA-Lösung versetzt. Anschließend dosiert man bei 38 °C bis Rückflusstemperatur (64-65 °C) 60 ml einer 25%igen wässrigen Ammoniaklösung zu. Dabei beginnt das Tosylat auszufallen. Es wird auf RT abgekühlt und weitere 20 ml einer 25%igen wässrigen Ammoniaklösung werden zugegeben. Das Produkt **1** wird abfiltriert und mit Wasser nachgewaschen. Die Trocknung erfolgt bei 60 °C oder bis zu 95 °C im Vakuum. Ausbeute: 42,4 g (89,3 %); Reinheit: > 99 % HPLC-Peakfläche

## Patentansprüche

1. Verfahren zur Herstellung der Verbindung der Formel **1** 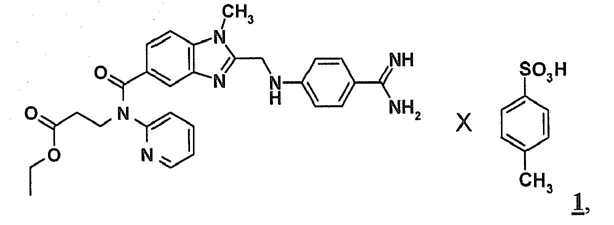 **dadurch gekennzeichnet, dass** ein Diamin der Formel **2** 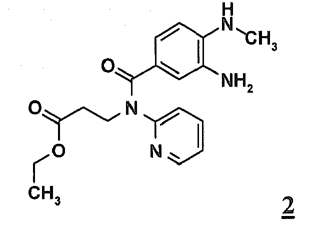 durch Umsetzung mit einem Oxadiazolon der Formel **3** 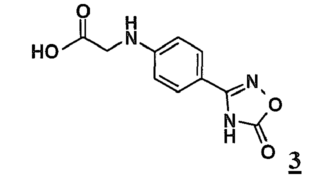 zu einer Verbindung der Formel **4** 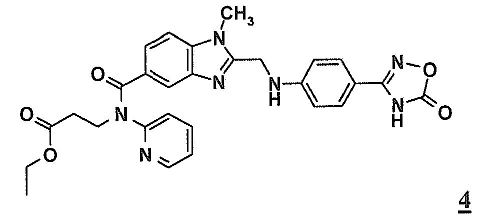 umgesetzt wird, die ohne Isolierung durch Hydrierung und Zugabe von p-Toluolsulfonsäure und Ammoniak in das Amidin der Formel **1** umgewandelt wird.

2. Verfahren nach Anspruch **1**, **dadurch gekennzeichnet, dass** die Umsetzung von **2** und **3** zur Zwischenverbindung **4** in einem inerten, organischen Lösungsmittel in Gegenwart eines wasserbindenden Mittels erfolgt.

3. Verfahren nach Anspruch 2, **dadurch gekennzeichnet, dass** das Lösungsmittel ein aprotische Lösungsmittel darstellt, dass bevorzugt ausgewählt ist aus der Gruppe bestehend aus aliphatische oder aromatische, gegebenenfalls halogenierte Kohlenwasserstoffe, Ether, Amide oder Gemische davon.

4. Verfahren nach Anspruch 2 oder 3, **dadurch gekennzeichnet, dass** das wasserbindende Mittel ausgewählt ist aus hygroskopischen Salzen, anorganischen Säuren, organischen Säuren, orangischen Säurechloriden, Anhydriden von anorganischen Säuren, Anhydriden von organischen Säuren, Anhydriden von Alkanphosphonsäuren, Molsieben, Harnstoffderivaten und Alkanphosphonsäureanhydriden.

5. Verfahren nach Anspruch 4, **dadurch gekennzeichnet, dass** das wasserbindende Mittel ausgewählt ist aus der Gruppe bestehend aus 1,1 '-Carbonyldiimidazol und Propanphosphonsäureanhydrid.

## Claims

1. Process for preparing the compound of formula **1** 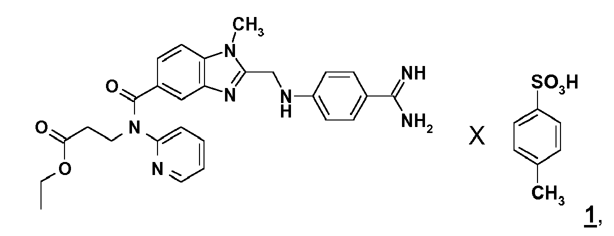 **characterised in that** a diamine of formula **2** 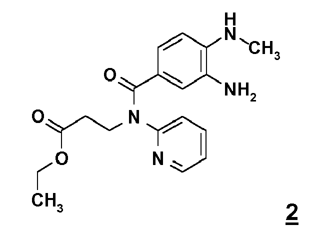 is reacted, by reaction with an oxadiazolone of formula **3** 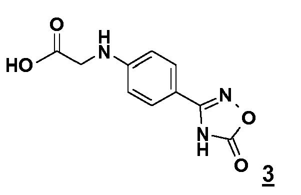 to form a compound of formula **4** 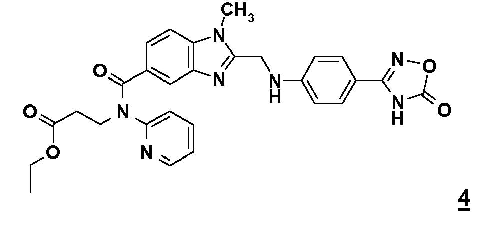 which, without being isolated, is converted into the amidine of formula **1** by hydrogenation and addition of p-toluenesulphonic acid and ammonia.

2. Process according to claim 1, **characterised in that** the reaction of **2** and **3** to obtain the intermediate compound **4** is carried out in an inert organic solvent in the presence of a water-binding agent.

3. Process according to claim 2, **characterised in that** the solvent is an aprotic solvent that is preferably selected from among aliphatic or aromatic, optionally halogenated hydrocarbons, ethers, amides or mixtures thereof.

4. Process according to claim 2 or 3, **characterised in that** the water-binding agent is selected from among hygroscopic salts, inorganic acids, organic acids, organic acid chlorides, anhydrides of inorganic acids, anhydrides of organic acids, anhydrides of alkanephosphonic acids, molecular sieves, urea derivatives and alkanephosphonic anhydrides.

5. Process according to claim 4, **characterised in that** the water-binding agent is selected from among 1,1'-carbonyldiimidazole and propanephosphonic anhydride.

## Revendications

1. Procédé de production du composé de formule 1 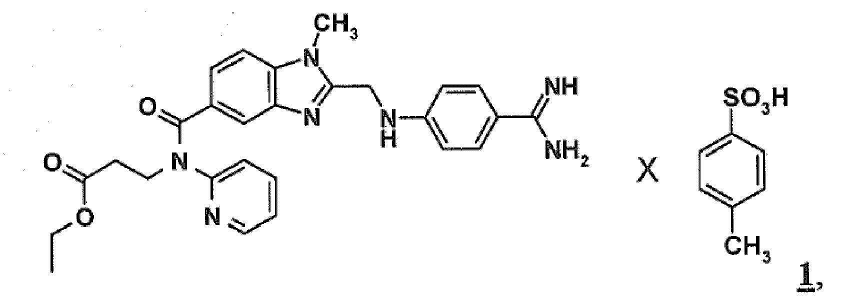 **caractérisé en ce que** l'on fait réagir une diamine de formule 2 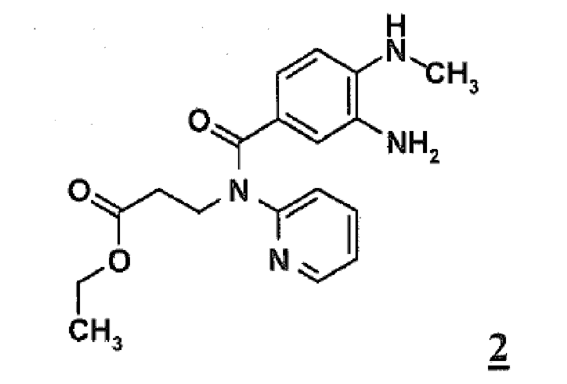 par réaction avec une oxadiazolone de formule 3 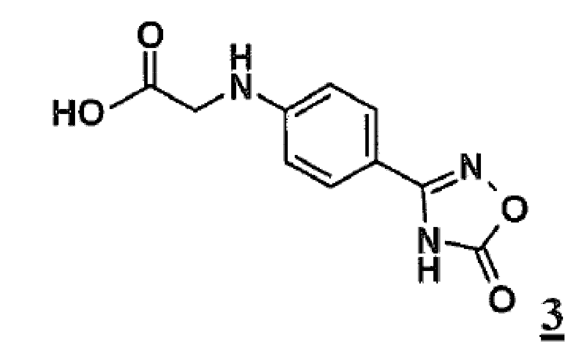 en un composé de formule 4 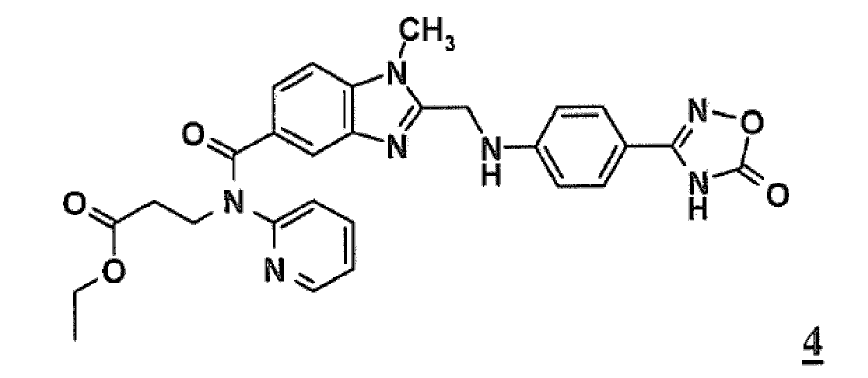 qui est converti sans isolement par hydrogénation et addition d'acide p-toluène-sulfonique et d'ammoniaque en l'amidine de formule 1.

2. Procédé selon la revendication 1, **caractérisé en ce que** la réaction des composés 2 et 3 en composé intermédiaire 4 s'effectue dans un solvant inerte organique en présence d'un agent fixant l'eau.

3. Procédé selon la revendication 2, **caractérisé en ce que** le solvant est un solvant aprotique qui est choisi de préférence dans le groupe comprenant des hydrocarbures, des éthers, des amides aliphatiques ou aromatiques, éventuellement halogénés, ou des mélanges de ceux-ci.

4. Procédé selon la revendication 2 ou 3, **caractérisé en ce que** l'agent fixant l'eau est choisi parmi des sels hygroscopiques, des acides inorganiques, des acides organiques, des chlorures d'acide organiques, des anhydrides d'acides inorganiques, des anhydrides d'acides organiques, des anhydrides d'acides alcane-phosphonique, des tamis moléculaires, des dérivés d'urée et des anhydrides d'acide alcanephosphonique.

5. Procédé selon la revendication 4, **caractérisé en ce que** l'agent fixant l'eau est choisi dans le groupe comprenant le 1,1'-carbonyldiimidazole et l'anhydride d'acide propanephosphonique.
